# FASCICULE DE BREVET EUROPEEN

(11) **EP 3 063 255 B1**
(45) Date de publication et mention de la délivrance du brevet: **09.01.2019**
(21) Numéro de dépôt: 14824894.1
(22) Date de dépôt: 28.10.2014
(51) Int. Cl.: C10J 3/00, C10L 3/08, C07C 1/04, C07C 1/12, C10J 3/12, C10J 3/82, C10J 3/84, B01J 8/04

(54) **DISPOSITIF ET PROCÉDÉ DE PRODUCTION DE GAZ NATUREL DE SUBSTITUT ET RESEAU LE COMPORTANT**
VORRICHTUNG UND VERFAHREN ZUR HERSTELLUNG VON SYNTHETISCHEM ERDGAS UND NETZWERK DAMIT
DEVICE AND METHOD FOR PRODUCING SUBSTITUTE NATURAL GAS AND NETWORK COMPRISING SAME

(30) Priorité: 28.10.2013 FR 1360488
(43) Date de publication de la demande: 07.09.2016
(73) Titulaire: GDF SUEZ, 92400 Courbevoie (FR)
(72) Inventeur: KARA, Yilmaz, F-95600 Eaubonne (FR); MARCHAND, Bernard, F-75018 Paris (FR); CAPELA, Sandra, F-93500 Pantin (FR)
(74) Mandataire: Cornuejols, Georges
(86) Numéro de dépôt international: PCT/FR2014/052745
(87) Numéro de publication internationale: WO 2015/063411

(56) Documents cités:
- EP-A2- 2 505 632
- FR-A1- 2 982 857
- M Saric ET AL: "Power-to-Gas coupling to biomethane production", ICPS 13 International Conference on Polygeneration Strategies, 1 septembre 2013 (2013-09-01), XP055122170, Vienna, Austria Extrait de l'Internet: URL:http://www.edgar-program.com/uploads/f ckconnector/cdbb0f9d-9e4f-4dbf-add6-13edc6 754fe3 [extrait le 2014-06-06]
- "Research for global markets for renewable energies", , 1 avril 2011 (2011-04-01), XP055122218, Berlin Extrait de l'Internet: URL:https://web.archive.org/web/2011092317 1659/http://www.fvee.de/fileadmin/publikat ionen/Themenhefte/th2009-1/th2009-1.pdf [extrait le 2014-06-06]

## Description

### DOMAINE TECHNIQUE DE L'INVENTION

La présente invention vise un dispositif et procédé de production de gaz naturel de substitution et un réseau le comportant. Elle s'applique notamment à la méthanation industrielle et à la cogénération d'énergie thermique et de méthane.

### ETAT DE LA TECHNIQUE

La méthanation est un procédé industriel de conversion catalytique de l'hydrogène et du monoxyde ou du dioxyde de carbone en méthane.

Selon la nature du composé à base de carbone, la formule de la réaction de méthanation change. Cette formule est, selon le cas :

CO + 3 H₂ → CH₄ + H₂O

CO₂ + 4 H₂ → CH₄ + 2 H₂O

Habituellement, un dispositif de production de biométhane dont l'entrée principale est une biomasse comporte trois éléments principaux. Le premier élément est un moyen de gazéification de la biomasse en gaz synthétique (aussi appelé « syngas »). Ce syngas est composé essentiellement de gaz incondensables, comme par exemple du H₂, CO, CO₂ ou CH₄. Pour certains procédés, en plus du syngas produits, le moyen de gazéification produit, de plus, des gaz condensables de type goudrons, ci-après appelés « tars », et des résidus solides de type « chars », c'est à dire une partie solide résultant d'une pyrolyse d'un combustible solide.

Le moyen de gazéification est associé à un moyen de combustion dans lequel les résidus solides, comme les chars, sont brûlés pour assurer le maintien en température du moyen de gazéification. Ce moyen de combustion est habituellement un réacteur à lit transporté ou circulant. Ce média fluidisé est préférentiellement constitué de particules d'olivine catalyseur et plus généralement d'un solide caloporteur comme du sable par exemple. Ce média fluidisé permet de faciliter le soutirage de chars résiduels n'ayant pas réagis dans le moyen de gazéification et de faciliter l'acheminement de ces chars vers le moyen de combustion.

Le deuxième élément principal est la méthanation catalytique de la biomasse gazéifiée, cette méthanation consistant à convertir le H₂ et le CO en CH₄ (SNG pour « Synthetic Natural Gas », gaz naturel synthétique en anglais).

Le troisième élément principal est la mise aux spécifications du SNG résiduel, c'est à dire l'élimination du H₂, CO, H₂O et CO₂ résiduel afin de produire un SNG au plus proche des spécifications d'injection sur le réseau de gaz naturel, en particulier en terme de pouvoir calorifique supérieur, dit « PCS » et d'indice de Wobbe. Pour rappel, l'indice de Wobbe permet d'évaluer la capacité d'interchangeabilité entre des gaz, carburant ou combustibles.

M. Saric et al.: « Power-to-Gas coupling to biomethane production », publié le 1er septembre 2013 décrit l'utilisation d'un réseau de gaz naturel comme moyen de stockage de l'excès d'électricité pat production de méthane de synthèse.

Le principal inconvénient des systèmes actuels provient de l'absence d'optimisation dans le rendement de SNG en sortie du système en raison des nombreuses pertes de carbone et d'énergie tout au long de la chaine décrite ci-dessus.

### OBJET DE L'INVENTION

La présente invention vise à remédier à tout ou partie de ces inconvénients.

A cet effet, la présente invention vise, selon un premier aspect, un dispositif intégré de production de gaz naturel de substitution, qui comporte :
- un gazéificateur configuré pour produire un composé gazeux à partir d'une biomasse, comportant :
   - une entrée pour la biomasse ;
   - une entrée pour un agent oxydant et
   - une sortie du composé gazeux comportant du monoxyde de carbone ;
   - un premier moyen de méthanation du monoxyde de carbone, disposé en aval du gazéificateur, pour produire un composé comportant du gaz naturel de substitution à partir du composé gazeux sortant du gazéificateur, le moyen de méthanation du monoxyde de carbone comportant au moins une entrée pour de l'eau et une entrée pour le composé gazeux issu du gazéificateur,
- en aval du premier moyen de méthanation de monoxyde de carbone, un séparateur de dioxyde de carbone pour alimenter un deuxième moyen de méthanation de dioxyde de carbone en dioxyde de carbone,
- le deuxième moyen de méthanation de dioxyde de carbone pour produire un composé comportant du gaz naturel de substitution comportant au moins une entrée pour de l'eau et une entrée pour du dioxyde de carbone provenant du séparateur,
- un moyen de production de dihydrogène à partir d'eau et de courant électrique comportant :
   - une alimentation électrique,
   - une entrée pour eau et
   - une sortie pour dihydrogène alimentant le deuxième moyen de méthanation de dioxyde de carbone

On note que « gazéificateur » est par abus de langage parfois appelé « gazéifieur » (de l'anglais « gazeifier »).

Grâce à ces dispositions, le dioxyde de carbone présent en sortie du moyen de méthanation de monoxyde de carbone est transformé en SNG par le biais du moyen de méthanation de dioxyde de carbone, augmentant ainsi le rendement de conversion du carbone du dispositif dans son ensemble. De plus, la présence d'un moyen d'électrolyse de l'eau permet de réaliser des applications de type « power to gas » (traduit en français par « énergie vers gaz »). Pour rappel, les applications de type power to gas consistent à convertir de l'énergie électrique inutilisée, par exemple produites de nuit par une centrale nucléaire, en gaz de substitution pouvant être consommé ultérieurement pour régénérer de l'énergie électrique.

Dans des modes de réalisation, le dispositif objet de la présente invention comporte, de plus, un moyen de combustion, comportant :
- une entrée pour une partie solide résultant d'une pyrolyse d'un combustible solide, aussi appelée « char », non gazéifié en provenance du gazéificateur et transporté par un média caloporteur,
- une entrée pour comburant,
- un moyen de combustion du char non gazéifié pour chauffer le média caloporteur,
- une sortie du média caloporteur reliée à une entrée pour média caloporteur du gazéificateur et
- une sortie pour fumées.

L'avantage de ces modes de réalisations est qu'ils permettent d'augmenter le rendement du gazéificateur en utilisant des rejets carbonés non gazéifiés afin de générer de la chaleur chauffant le gazéificateur. La combustion de ces rejets carbonés permettant, de plus, de chauffer le média caloporteur transportant les rejets carbonés.

Dans des modes de réalisation, le moyen de production de dihydrogène est configuré pour réaliser une électrolyse de l'eau comportant une sortie pour dioxygène alimentant l'entrée en comburant du moyen de combustion.

Ces modes de réalisation ont l'avantage d'augmenter drastiquement le rendement en gaz naturel de substitution en permettant d'éviter d'injecter une partie du gaz synthétique sorti du gazéificateur dans le moyen de combustion afin de rendre la combustion possible. En particulier, ces modes de réalisation permettent de maximiser l'efficacité d'une application power to gas en utilisant l'ensemble des produits de l'électrolyse de l'eau et en optimisant le rendement en gaz naturel de substitution.

Dans des modes de réalisation, le dispositif objet de la présente invention comporte, entre la sortie de composé gazeux du gazéificateur et l'entrée pour composé gazeux du moyen de méthanation de monoxyde de carbone, un séparateur configuré pour séparer les gaz des solides et/ou goudrons dans le composé gazeux et pour transmettre les solides et/ou goudrons séparés au moyen de combustion.

Le premier avantage de ces modes de réalisation est qu'ils permettent d'épurer le gaz synthétique sorti du gazéificateur en ôtant des solides pouvant être charriés avec le gaz. Le deuxième avantage de ces modes de réalisation est qu'ils permettent de recycler les solides en les utilisant dans le moyen de combustion, augmentant ainsi le rendement du moyen de combustion.

Dans des modes de réalisation, le dispositif objet de la présente invention comporte un moyen de recyclage d'une partie de la fumée, en sortie du moyen de combustion, comportant du dioxygène, vers une entrée de comburant du moyen de combustion.

Ces modes de réalisation permettent d'augmenter le rendement du moyen de combustion en recyclant une partie des produits du moyen de combustion. Ces modes de réalisation permettent, pour un équipement donné, de pouvoir fonctionner aussi bien en aérocombustion qu'en oxycombustion. Pour un procédé initialement conçu pour fonctionner en aérocombustion, le fait de passer en oxycombustion entraine une chute drastique des vitesses et conduit à l'arrêt de la circulation du solide caloporteur et donc de la production de gaz. Dans ce cas, pour passer en oxycombustion, il faut soit un nouveau moyen de combustion avec un diamètre plus faible pour avoir des vitesses de transport suffisantes, soit une recirculation de fumée pour compenser l'absence de diazote dans le comburant. Le choix des fumées est certainement le plus pertinent car c'est un produit issu du même système.

Dans des modes de réalisation, le dispositif objet de la présente invention comporte, en aval de la sortie de fumée du moyen de combustion, un séparateur de dioxyde de carbone configuré pour alimenter le moyen de méthanation de dioxyde de carbone en dioxyde de carbone.

Ces modes de réalisation permettent d'augmenter le rendement du moyen de méthanation de dioxyde de carbone.

Dans des modes de réalisation, le dispositif objet de la présente invention comporte un séparateur de dihydrogène en aval du moyen de méthanation de monoxyde de carbone pour alimenter ledit moyen de méthanation de monoxyde de carbone en dihydrogène.

Ces modes de réalisation permettent d'augmenter le rendement du moyen de méthanation de monoxyde de carbone. Ces modes de réalisation sont préférentiels dans le cas où l'indice de Wobbe ou le pouvoir calorifique supérieur du gaz de synthèse n'est pas conforme aux exigences du réseau de transport de gaz auquel le gaz synthétique est alimenté.

Dans des modes de réalisation, le dispositif objet de la présente invention comporte en aval du moyen de méthanation de monoxyde de carbone, un séparateur de dioxyde de carbone pour alimenter le moyen de méthanation de dioxyde de carbone.

Ces modes de réalisation permettent de séparer le méthane en sortie du moyen de méthanation du monoxyde de carbone du dioxyde de carbone à alimenter au moyen de méthanation du dioxyde de carbone. Ainsi, le gaz en entrée du moyen de méthanation du dioxyde de carbone est plus concentré en dioxyde de carbone, augmentant de fait le rendement en sortie du moyen de méthanation de dioxyde de carbone.

Dans des modes de réalisation, une sortie du moyen de méthanation de dioxyde de carbone est reliée à une sortie du moyen de méthanation de monoxyde de carbone.

Ces modes de réalisation permettent de minimiser le nombre de dispositifs nécessaires entre les sorties de chaque moyen de méthanation et une sortie de gaz naturel de substitution du dispositif.

Dans des modes de réalisation, le dispositif objet de la présente invention comporte, en aval du moyen de méthanation de monoxyde de carbone et/ou du moyen de combustion, un condenseur configuré pour condenser de l'eau contenu dans des vapeurs et pour alimenter le moyen d'électrolyse en eau.

Ces modes de réalisation permettent d'augmenter les rendements du moyen d'électrolyse.

Selon un deuxième aspect, la présente invention vise un réseau, qui comporte au moins un dispositif objet de la présente invention.

Les buts, avantages et caractéristiques du réseau étant identiques à ceux du dispositif objet de la présente invention, ils ne sont pas rappelés ici.
Dans des modes de réalisation, le réseau objet de la présente invention comporte un comporte un moyen de gestion multi-énergie pour commander :
- la production, avec au moins un dispositif objet de la présente invention, et le stockage du méthane au cours de durées de production d'électricité en excès et
- la production d'électricité avec le méthane stocké en dehors de ces durées.

Ces modes de réalisation permettent d'optimiser la quantité d'énergie disponible dans le réseau pendant les durées où l'électricité produite n'est pas en excès.

Dans des modes de réalisation, le réseau objet de la présente invention comporte des canalisations de distribution de gaz, le stockage du méthane pour générer de l'électricité étant réalisé par surpression au-delà de la pression nominale des canalisations.

Ces modes de réalisation permettent de stocker à moindre coût le méthane produit par le dispositif objet de la présente invention.

Selon un troisième aspect, la présente invention vise un procédé de production de gaz naturel de substitution, qui comporte :
- une étape de gazéification pour produire un composé gazeux à partir d'une biomasse, comportant :
   - une étape d'entrée de la biomasse ;
   - une étape d'entrée d'un agent oxydant et
   - une étape de sortie du composé gazeux comportant du monoxyde de carbone ;
   - une première étape de méthanation du monoxyde de carbone, en aval de l'étape de gazéification, pour produire un composé comportant du gaz naturel de substitution à partir du composé gazeux issu de l'étape de gazéification, l'étape de méthanation du monoxyde de carbone comportant au moins une étape d'entrée pour de l'eau et pour le composé gazeux issu du gazéificateur,
- en aval de la première étape de méthanation de monoxyde de carbone, une étape de séparation de dioxyde de carbone pour alimenter une deuxième étape de méthanation de dioxyde de carbone en dioxyde de carbone,
- une deuxième étape de méthanation de dioxyde de carbone pour produire un composé comportant du gaz naturel de substitution comportant au moins une étape d'entrée pour de l'eau et une entrée pour du dioxyde de carbone provenant de l'étape de séparation,
- une étape de production de dihydrogène à partir d'eau et de courant électrique comportant :
   - une étape d'alimentation électrique,
   - une étape d'entrée pour eau et
   - une étape de sortie pour dihydrogène utilisé au cours de l'étape de méthanation de dioxyde de carbone.

Les buts, avantages et caractéristiques du réseau étant identiques à ceux du dispositif objet de la présente invention, ils ne sont pas rappelés ici.

### BREVE DESCRIPTION DES FIGURES

D'autres avantages, buts et caractéristiques particulières de l'invention ressortiront de la description non limitative qui suit d'au moins un mode de réalisation particulier du dispositif et procédé de production de gaz naturel de substitution et du réseau comportant ledit dispositif objets de la présente invention, en regard des dessins annexés, dans lesquels :
- la figure 1 représente, schématiquement, un mode de réalisation particulier du dispositif de production de gaz naturel de substitution objet de la présente invention,
- la figure 2 représente, schématiquement, un mode de réalisation particulier du réseau objet de la présente invention et
- la figure 3 représente un logigramme d'étapes d'un mode de réalisation particulier du procédé objet de la présente invention.

### DESCRIPTION D'EXEMPLES DE REALISATION DE L'INVENTION

La présente description est donnée à titre non limitatif.

On note, dès à présent, que les figures ne sont pas à l'échelle.

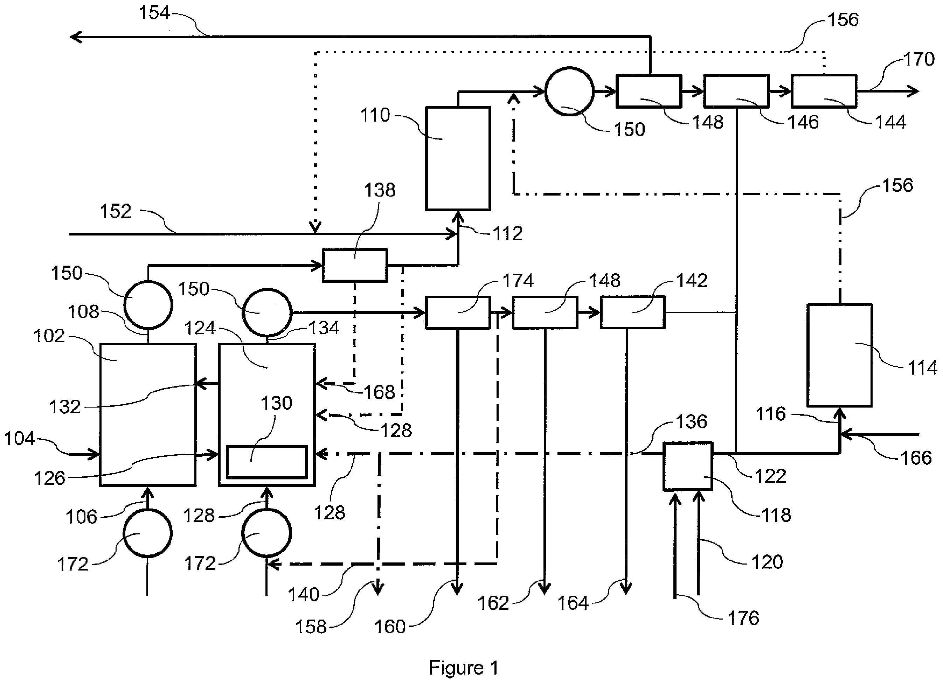

On observe, sur la figure 1, un mode de réalisation du dispositif intégré de production de gaz naturel de substitution objet de la présente invention. Ce dispositif comporte :
- un gazéificateur 102, comportant :
   - une entrée 104 pour biomasse ;
   - une entrée 106 pour un agent oxydant et
   - une sortie 108 pour gaz synthétique comportant du monoxyde de carbone ;
- un séparateur 138 configuré pour transmettre des solides et goudrons séparés au moyen 124 de combustion,
- un moyen 110 de méthanation du monoxyde de carbone sortant du gazéificateur 102 comportant au moins une entrée 112 pour de l'eau et pour gaz synthétique issu du gazéificateur 102, fournissant du méthane et du dioxyde de carbone,
- un séparateur 144 de dihydrogène,
- un premier séparateur 146 de dioxyde de carbone,
- un moyen 114 de méthanation du dioxyde de carbone comportant au moins une entrée 116 pour de l'eau et pour dioxyde de carbone provenant du moyen de méthanation de monoxyde de carbone, fournissant du méthane,
- un moyen 118 d'électrolyse d'eau comportant :
   - une alimentation 176 électrique ;
   - une entrée 120 pour eau ;
   - une sortie 136 pour dioxygène et
   - une sortie 122 pour dihydrogène et
- un moyen 124 de combustion, comportant :
   - une entrée 126 pour char non gazéifié transporté par un média caloporteur en provenance du gazéificateur 102 ;
   - trois entrées 128 ;
   - un moyen 130 de combustion du char non gazéifié, de goudrons et de syngas d'appoint pour chauffer le média caloporteur ;
   - une sortie 132 du média caloporteur reliée à une entrée pour média caloporteur du gazéificateur 102 et
   - une sortie 134 pour fumées
   - une entrée 168 de char non gazéifié et pour goudrons séparés du gaz sorti du gazéificateur 102 et
- un moyen 140 de recyclage d'une partie de la fumée en sortie du moyen 124 de combustion,
- un deuxième séparateur 142 de dioxyde de carbone et
- deux condenseurs 148,
- trois moyens 150 de refroidissement,
- deux moyens 172 de chauffage,
- une première entrée 152 de vapeur d'eau,
- une sortie 158 de dioxygène,
- une sortie 160 de cendres et résidus solides,
- une première sortie 154 d'eau,
- une deuxième sortie 162 d'eau,
- une sortie 164 de gaz inutilisés par le dispositif,
- une deuxième entrée 166 de vapeur d'eau et
- une sortie 170 pour gaz naturel de substitution.

Le gazéificateur 102 est, par exemple, un réacteur dans lequel la biomasse alimentée subit une conversion thermochimique pour former un gaz synthétique (aussi appelé « syngas ») contenant du dihydrogène, du monoxyde de carbone, du dioxyde de carbone, de l'eau, des goudrons ou de manière général tout type de composé carboné. Ce gazéificateur 102 comporte une entrée 104 pour biomasse qui est, par exemple, une vanne, une vis doseuse ou une trémie permettant d'introduire de la biomasse dans le réacteur. Ce gazéificateur 102 comporte de plus une entrée 106 pour un agent oxydant qui est, par exemple, une vanne permettant d'introduire de la vapeur d'eau dans le réacteur. En amont de cette entrée 106 pour agent oxydant, un moyen 172 de chauffage est placé afin que l'oxydant entrant ne perturbe pas l'équilibre thermique à l'intérieur du gazéificateur 102.

Le gazéificateur 102 comporte, de plus, une sortie (non représentée) pour char non gazéifié qui est, par exemple, une conduite dans laquelle est transférée un média fluidisé caloporteur. Ce média fluidisé caloporteur est, par exemple, fait d'olivine ou de sable et fournit l'énergie nécessaire à la conversion thermochimique de la biomasse. Ce gazéificateur 102 comporte également une entrée, non représentée, pour média fluidisé caloporteur. Ce gazéificateur 102 comporte enfin une sortie 108 pour gaz synthétique qui est, par exemple, une conduite connectée au réacteur.

Afin de chauffer le gazéificateur 102, le dispositif comporte un moyen 124 de combustion. Ce moyen 124 de combustion est, par exemple, un réacteur. Ce moyen 124 de combustion comporte une entrée 126 pour char non gazéifié transporté par un média caloporteur en provenance du gazéificateur 102 qui est, par exemple, une conduite reliant le gazéificateur 102 au moyen 124 de combustion. Ce moyen 124 de combustion comporte également trois entrées 128 pour comburant qui sont, par exemple, des vannes reliées à des conduites permettant d'introduire du comburant dans le moyen 124 de combustion. Une entrée 128 est configurée pour insérer dans le moyen 124 de combustion de l'air, de l'azote ou du dioxygène ou le mélange de l'ensemble, comme par exemple de l'air enrichie en dioxygène. On place, en amont de cette entrée 128, un moyen 172 de chauffage, optionnel, du comburant de manière à ce que le comburant entré ne perturbe pas l'équilibre thermique interne du moyen 124 de combustion. Une autre entrée 128 est configurée pour insérer dans le moyen 124 de combustion du dioxygène provenant de l'électrolyse de l'eau. La dernière entrée 128 est configurée pour insérer si nécessaire en tant qu'appoint thermique dans le cas où char et goudrons ne suffisent pas, dans le moyen 124 de combustion de gaz synthétique issu du gazéificateur 102.

Dans des variantes, ces entrées 128 de comburant peuvent être combinées en deux ou une seule entrée de comburant. Le moyen 124 de combustion réalise la combustion du char non gazéifié et/ou des goudrons issus de l'entrée 168 afin de chauffer le média caloporteur, ce média caloporteur quittant le moyen 124 de combustion par le biais d'une sortie 132 du média caloporteur reliée à une entrée pour média caloporteur du gazéificateur 102 qui est, par exemple, une conduite reliant le moyen 124 de combustion et le gazéificateur 102. Le moyen 124 de combustion comporte, de plus, une sortie 134 pour fumées qui est par exemple une conduite connectée au moyen 124 de combustion.

L'utilisation de dioxygène comme comburant améliore le rendement énergétique du moyen 124 de combustion. L'utilisation du dioxygène permet notamment une réduction drastique du gaz synthétique en sortie du gazéificateur 102 à réutiliser comme comburant. Le dioxygène produit en excès par le moyen 118 d'électrolyse peut également être valorisé d'autres façons. Par ailleurs, plus la teneur en dioxygène dans le comburant est grande, plus l'efficacité de la chaine de séparation comportant le condenseur 148 et le séparateur de dioxyde de carbone est améliorée.

La composition du gaz synthétique généré par le gazéificateur 102 évolue sous l'action de la vapeur d'eau ou d'un autre agent oxydant, comme par exemple du dioxygène ou de l'air, entré dans le réacteur du fait des équilibres thermochimiques et de la production de composés par gazéification hétérogène de char. Pour cette raison, le gaz synthétique produit contient généralement des polluants néfastes à la durée de vie d'un catalyseur contenu dans le moyen 110 de méthanation du monoxyde de carbone. Pour cette raison, un moyen 150 de refroidissement, ou de récupération de chaleur, est placé en sortie du gazéificateur 102 et, à la sortie de ce moyen 150 de refroidissement, un séparateur 138 configuré pour transmettre les solides et goudrons séparés au moyen 124 de combustion. Ce moyen 150 de refroidissement est, par exemple, un échangeur thermique. Ce moyen 150 de refroidissement permet de réaliser un échange de chaleur, la chaleur étant récupérée pour être valorisée ailleurs dans le dispositif.

Le séparateur 138 est, par exemple, un filtre configuré pour retenir les composés solides couplé à une absorption pour retenir les goudrons. Ce séparateur 138 alimente le moyen 124 de combustion des solides ainsi séparés par le biais, par exemple, d'une conduite. Les solides ainsi retenus peuvent être des composés organiques, inorganiques comme des tars, du sulfure d'hydrogène, du sulfure de monoxyde de carbone ou bien une grande partie de l'eau et des solides transportés avec le flux gazeux. Une partie du gaz à l'issue du séparateur 138 peut être alimentée, au besoin, au moyen 124 de combustion.

De même, la fumée en sortie du moyen 124 de combustion est traitée de manière analogue par le biais d'un moyen 150 de refroidissement, ou de récupération de chaleur, comme par exemple un échangeur thermique, refroidissant les fumées et d'un séparateur 174 gaz/solides configuré pour transférer les solides filtrés à une sortie 160 de cendres et de solides élutriés. Une partie du gaz, contenant du dioxygène, en sortie de ce séparateur 174 peut être alimenté, au besoin, au moyen 124 de combustion en guise de comburant.

Le dispositif comporte un moyen 110 de méthanation du monoxyde de carbone sortant du gazéificateur 102 qui est, par exemple, un réacteur catalytique de méthanation. Ce réacteur catalytique de méthanation est, par exemple, un réacteur à lit fixe, à lit fluidisé ou de type réacteurs-échangeurs. Ce réacteur catalytique de méthanation transforme du monoxyde de carbone, du dihydrogène et de l'eau en dioxyde de carbone et méthane. Ce moyen 110 de méthanation de monoxyde de carbone comporte une entrée 112 pour de l'eau et pour gaz synthétique issu du gazéificateur 102. Cette entrée 112 est par exemple une vanne permettant d'insérer dans le moyen 110 de méthanation de monoxyde de carbone de la vapeur d'eau et du gaz synthétique.

La vapeur d'eau entre dans le dispositif par le biais d'une première entrée 152 d'eau qui alimente l'entrée 112 pour de l'eau et pour gaz synthétique. L'ajout de vapeur d'eau permet d'assurer l'ajustement du ratio dihydrogène sur monoxyde de carbone proche de la stoechiométrie par la réaction de water gas shift (CO + H₂O = H₂ + CO₂) et ainsi éviter une désactivation prématurée du catalyseur par dépôt de coke. Le moyen 110 de méthanation de monoxyde de carbone produit, en sortie, du méthane et du dioxyde de carbone.

Le mélange gazeux en sortie du moyen 110 de méthanation est refroidit par un moyen 150 de refroidissement qui est, par exemple, un échangeur thermique. Le gaz synthétique en sortie est déshydraté par un condenseur 148. Ce condenseur 148 peut employer toutes les techniques de réduction d'eau ou leurs associations, comme par exemple la condensation thermique, l'adsorption ou l'absorption. L'eau ainsi récupérée est transmise à une sortie 154 d'eau. L'eau ainsi sortie peut être évacuée du dispositif ou être alimentée au moyen 118 d'électrolyse.

Le mélange gazeux en sortie du condenseur 148 est injecté dans un séparateur 146 de dioxyde de carbone. Le séparateur 146 de dioxyde de carbone peut utiliser toutes les méthodes connues ou leurs combinaisons, comme par exemple l'utilisation de la cryogénie, l'absorption ou l'adsorption. L'homme du métier retiendra la solution de son choix pour peu que cette solution permette d'obtenir du dioxyde de carbone avec une pureté supérieure à 85% en volume. Un volume trop important de monoxyde de carbone présent avec le dioxyde de carbone favorise la réaction de méthanation du monoxyde de carbone au détriment de la réaction de méthanation du dioxyde de carbone dans un réacteur 114 de méthanation.

Dans des variantes, le dioxyde de carbone récupéré est traité par un moyen d'épuration complémentaire configuré pour éliminer le monoxyde de carbone présent avec le dioxyde de carbone. Au delà des solutions classiques, comme par exemple l'adsorption ou l'absorption, le mélange contenant le dioxyde de carbone séparé par le séparateur 146 peut subir une oxydation thermique dans le moyen 124 de combustion. Il est à noter que l'oxydation thermique n'est envisageable que si le moyen 124 de combustion fonctionne au dioxygène pur ou si le dispositif comporte un séparateur de dioxyde de carbone en sortie du moyen 124 de combustion.

Dans d'autres variantes, le dispositif comporte un moyen de méthanation de finition du monoxyde de carbone en amont du moyen 114 de méthanation du dioxyde de carbone.

Le dispositif comporte un moyen 114 de méthanation du dioxyde de carbone sortant du gazéificateur 102 qui est, par exemple, un réacteur catalytique de méthanation. Ce réacteur catalytique de méthanation est, par exemple, un réacteur à lit fixe, à lit fluidisé ou de type réacteurs-échangeurs. Ce réacteur catalytique de méthanation transforme du dioxyde de carbone, du dihydrogène et de l'eau en dioxyde de carbone et méthane. Ce moyen 114 de méthanation de dioxyde de carbone comporte une entrée 116 pour de l'eau et pour gaz synthétique issu du séparateur 146. Cette entrée 116 est par exemple une vanne permettant d'insérer dans le moyen 114 de méthanation de dioxyde de carbone de la vapeur d'eau et du gaz synthétique. La vapeur d'eau entre dans le dispositif par le biais d'une première entrée 166 d'eau qui alimente l'entrée 116 pour de l'eau et pour gaz synthétique. Le moyen 114 de méthanation de dioxyde de carbone produit, en sortie, du méthane et de l'eau.

En complément du dioxyde de carbone séparé en sortie du moyen 110 de méthanation du monoxyde de carbone, du dioxyde de carbone est récupéré des fumées en sortie du moyen 124 de méthanation. Pour ce faire, le dispositif comporte en sortie du séparateur 174 gaz-solide en sortie du moyen 124 de méthanation un condenseur 148 configuré pour déshydrater la fumée sortie du séparateur 174. L'eau récupérée est transférée à une sortie 162 d'eau permettant l'évacuation de l'eau du dispositif ou le transfert de cette eau vers le moyen 118 d'électrolyse de l'eau.

En sortie de ce condenseur 148, le mélange gazeux restant entre dans un séparateur 142 de dioxyde de carbone similaire au séparateur 146 de dioxyde de carbone en sortie du moyen 110 de méthanation du monoxyde de carbone. Les gaz séparés du dioxyde de carbone sont alimentés à une sortie 164 de gaz inutilisés par le dispositif. Le dioxyde de carbone séparé par le séparateur 142 est alimenté en entrée du moyen 114 de méthanation du dioxyde de carbone.

La sortie 156 de méthane et d'eau du moyen 114 de méthanation de dioxyde de carbone est connectée à la sortie, non représentée, du moyen 110 de méthanation de monoxyde de carbone, en aval du moyen 150 de refroidissement.

En aval du séparateur 146 de dioxyde de carbone, le dispositif comporte un séparateur 144 de dihydrogène. Ce séparateur 144 de dihydrogène permet une mise aux spécifications du gaz synthétique aux caractéristiques du gaz naturel. Ce séparateur 144 de dihydrogène peut employer l'ensemble des méthodes usuelles ou leur combinaison. Le dihydrogène séparé est alimenté en entrée du moyen 110 de méthanation du monoxyde de carbone par le biais d'une conduite 156.

Le gaz synthétique en sortie du séparateur 144 de dihydrogène est alimenté à une sortie 170 de gaz synthétique du dispositif.

Le dispositif comporte un moyen 118 d'électrolyse d'eau configuré pour transformer de l'eau en dioxygène et en dihydrogène. Ce moyen 118 d'électrolyse est, par exemple, une cellule électrolytique comportant deux électrodes immergées dans l'eau chacune connectée à un pôle opposé d'une source 176 de courant continu. Ce moyen 118 d'électrolyse comporte une entrée 120 pour eau qui est, par exemple, une vanne permettant l'injection d'eau dans le moyen 118 d'électrolyse. Ce moyen 118 d'électrolyse comporte également une sortie 122 pour dihydrogène alimentant le moyen 114 de méthanation de dioxyde de carbone. De plus, ce moyen 118 d'électrolyse comporte une sortie 136 de dioxygène alimentant une entrée 128 pour comburant du moyen 124 de combustion. Ce dispositif comporte enfin une sortie 158 pour dioxygène pour évacuer les surplus de dioxygène du dispositif.

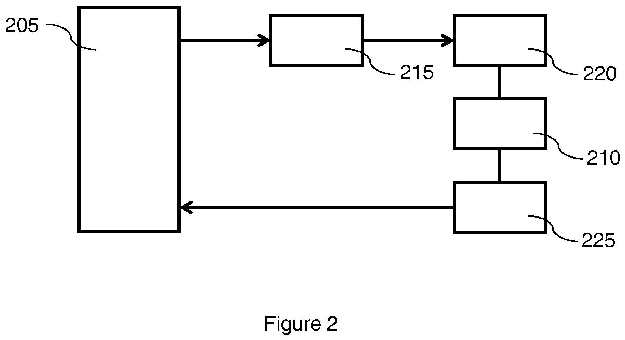

On observe, sur la figure 2, un mode de réalisation du réseau objet de la présente invention. Ce réseau comporte :
- un dispositif 205 de production de gaz naturel de substitution tel que décrit en figure 1,
- un moyen 210 de gestion multi-énergie,
- une canalisation 215 de transport ou distribution de gaz,
- un moyen 220 de conversion de gaz en électricité et
- un générateur 225 de courant continu.

Le moyen 210 de gestion multi-énergie est, par exemple, un commutateur qui commande :
- la production, par le dispositif 205, et le stockage du méthane au cours de durées de production d'électricité en excès et
- la production d'électricité avec le méthane stocké en dehors de ces durées.

Les durées de production d'électricité en excès peuvent être prédéterminées dans le système ou provenir d'une source d'information extérieure, comme un serveur par exemple.

Lorsque le moyen 210 de gestion multi-énergie identifie une durée de production d'électricité en excès, ce moyen 210 de gestion commande la production de méthane. Pour ce faire, de l'électricité en excès est utilisée par le générateur 225 de courant continu afin d'alimenter moyen d'électrolyse, non représenté, du dispositif 205 de production de gaz naturel de substitution. En parallèle, de la biomasse et un agent oxydant est inséré dans le gazéificateur du dispositif 205 de manière à produire du gaz synthétique. Le dispositif 205 produit, en sortie, du gaz naturel de substitution stocké par surpression, au delà de la pression nominale des canalisations, dans une canalisation 215 de distribution de gaz. Cette surpression est, par exemple, de l'ordre de 10%.

Lorsque le moyen 210 de gestion multi-énergie identifie une durée pendant laquelle la production d'énergie n'est pas en excès, ce moyen 210 de gestion commande la production d'électricité au moyen 220 de conversion de gaz en électricité. Le moyen 220 de conversion de gaz en électricité est, par exemple, une centrale thermique au gaz consommant le gaz naturel de substitution stocké par surpression dans la canalisation 215 afin de produire de l'électricité.

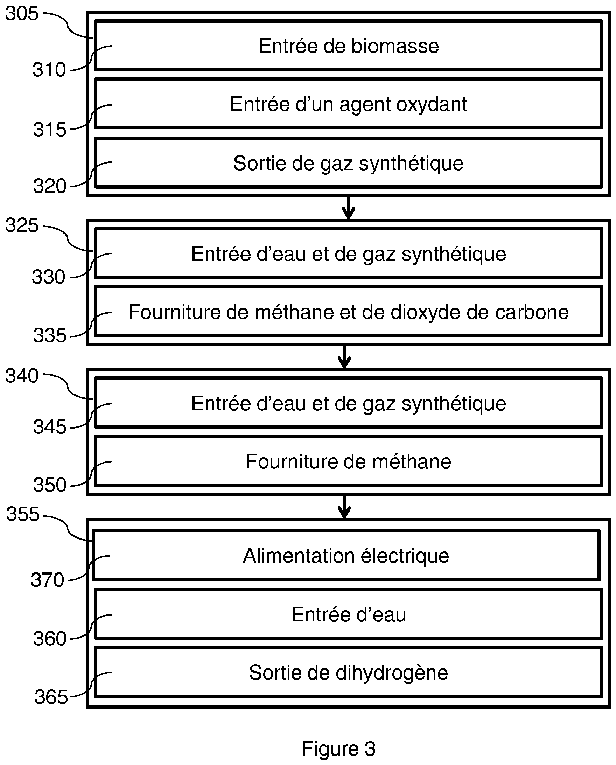

On observe, sur la figure 3, un logigramme d'étapes d'un mode de réalisation particulier du procédé objet de la présente invention. Ce procédé comporte :
- une étape 305 de gazéification pour produire un gaz synthétique, comportant :
   - une étape 310 d'entrée de biomasse ;
   - une étape 315 d'entrée d'un agent oxydant et
   - une étape 320 de sortie pour gaz synthétique comportant du monoxyde de carbone ;
- une étape 325 de méthanation du monoxyde de carbone issu de l'étape 305 de gazéification comportant une étape 330 d'entrée pour de l'eau et pour gaz synthétique issu de l'étape 305 de gazéification et une étape 335 de fourniture de méthane et de dioxyde de carbone,
- une étape 340 de méthanation du dioxyde de carbone comportant une étape 345 d'entrée pour de l'eau et pour dioxyde de carbone provenant de l'étape 325 de méthanation de monoxyde de carbone et une étape 350 de fourniture de méthane,
- une étape 355 d'électrolyse d'eau pour transformer de l'eau en dioxygène et en dihydrogène comportant :
   - une étape 370 d'alimentation électrique,
   - une étape 360 d'entrée pour eau et
   - une étape 365 de sortie pour dihydrogène utilisée au cours de l'étape 340 de méthanation de dioxyde de carbone.

L'étape 305 de gazéification est réalisée, par exemple, par la mise en oeuvre d'un gazéificateur qui est un réacteur dans lequel de la biomasse alimentée subit une conversion thermochimique pour former un gaz synthétique (aussi appelé « syngaz ») contenant du dihydrogène, du monoxyde de carbone, du dioxyde de carbone, de l'eau, des tars ou de manière général tout type de composé carboné.

L'étape 305 de gazéification comporte une étape 310 d'entrée de biomasse réalisée, par exemple, par la mise en oeuvre d'une vanne de fourniture de biomasse au gazéificateur. L'étape 305 de gazéification comporte également une étape 315 d'entrée d'un agent oxydant réalisée, par exemple, par la mise en oeuvre d'une vanne de fourniture d'agent oxydant au gazéificateur. L'étape 305 de gazéification comporte, de plus, une étape 320 de sortie pour gaz synthétique comportant du monoxyde de carbone réalisée, par exemple, par la mise en oeuvre d'une conduite connectée au gazéificateur.

Le procédé comporte une étape 325 de méthanation du monoxyde de carbone issu de l'étape 305 de gazéification réalisée, par exemple, par la mise en oeuvre d'un moyen de méthanation de monoxyde de carbone à lit fluidisé. Cette étape 325 de méthanation du monoxyde de carbone comporte une étape 330 d'entrée pour de l'eau et pour gaz synthétique issu de l'étape 305 de gazéification réalisée, par exemple, par la mise en oeuvre d'une vanne du moyen de méthanation. L'étape 325 de méthanation du monoxyde de carbone comporte étalement une étape 335 de fourniture de méthane et de dioxyde de carbone + H₂O réalisée, par exemple, par la mise en oeuvre d'une conduite en sortie du moyen de méthanation de monoxyde de carbone.

Le procédé comporte une étape 340 de méthanation du dioxyde de carbone réalisée, par exemple, par la mise en oeuvre d'un moyen de méthanation de dioxyde de carbone à lit fluidisé. L'étape 340 de méthanation du dioxyde de carbone comporte une étape 345 d'entrée pour de l'eau et pour dioxyde de carbone provenant de l'étape 325 de méthanation de monoxyde de carbone réalisée, par exemple, par la mise en oeuvre d'une vanne d'insertion d'eau et de dioxyde de carbone du moyen de méthanation de dioxyde de carbone. L'étape 340 de méthanation du dioxyde de carbone comporte une étape 350 de fourniture de méthane réalisée, par exemple, par la mise en oeuvre d'une conduite en sortie du moyen de méthanation du dioxyde de carbone.

Le procédé comporte une étape 355 d'électrolyse d'eau pour transformer de l'eau en dioxygène et en dihydrogène réalisée, par exemple, par la mise en oeuvre de deux électrodes plongées dans l'eau et connectée chacune à un pôle différent d'un générateur de courant continu. L'étape 355 d'électrolyse comporte une étape 360 d'entrée pour de l'eau réalisée, par exemple, par la mise en oeuvre d'une conduite d'injection d'eau entre les deux électrodes utilisées au cours de l'étape 355 d'électrolyse. L'étape 370 d'alimentation électrique est réalisée, par exemple, par la connexion des deux électrodes à une source de courant continu. L'étape 355 d'électrolyse comporte une étape 365 de sortie pour dihydrogène utilisée au cours de l'étape 340 de méthanation de dioxyde de carbone réalisée, par exemple, par la mise en oeuvre d'une conduite.

Dans des variantes, le procédé 30 comporte, de plus, une étape de combustion, comportant :
- une étape d'entrée entrée pour une partie solide résultant d'une pyrolyse d'un combustible solide, aussi appelée « char », non gazéifié en provenance du gazéificateur et transporté par un média caloporteur,
- une étape d'entrée pour comburant,
- une étape de combustion du char non gazéifié pour chauffer le média caloporteur,
- une étape de sortie du média caloporteur reliée à une entrée pour média caloporteur du gazéificateur et
- une étape de sortie pour fumées.

Dans des variantes, l'étape de production de dihydrogène réalise une électrolyse de l'eau comportant une étape de sortie pour dioxygène alimentant l'entrée en comburant d'un moyen de combustion mis en oeuvre au cours de l'étape de combustion.

Dans des variantes, le procédé 30 comporte, entre l'étape de sortie de composé gazeux du gazéificateur et l'étape d'entrée pour composé gazeux de l'étape de méthanation de monoxyde de carbone, une étape de séparation des gaz des solides et/ou goudrons dans le composé gazeux et une étape de transmission des solides et/ou goudrons séparés au moyen de combustion mis en oeuvre au cours de l'étape de combustion.

Dans des variantes, le procédé 30 comporte une étape de recyclage d'une partie de la fumée, en sortie de l'étape de combustion, comportant du dioxygène, vers une entrée de comburant du moyen de combustion mis en oeuvre au cours de l'étape de combustion.

Dans des variantes, le procédé 30 comporte, en aval de l'étape de sortie de fumée de l'étape de combustion, une étape de sépération de dioxyde de carbone alimenter le moyen de méthanation de dioxyde de carbone, mis en oeuvre par l'étape de méthanation de dioxyde de carbone, en dioxyde de carbone.

Dans des variantes, le procédé 30 comporte une étape de séparation de dihydrogène en aval de l'étape de méthanation de monoxyde de carbone pour alimenter le moyen de méthanation de monoxyde de carbone, mis en oeuvre au cours de l'étape de méthanation de monoxyde de carbone, en dihydrogène.

Le procédé 30 comporte en aval de l'étape de méthanation de monoxyde de carbone, une étape de séparation de dioxyde de carbone pour alimenter le moyen de méthanation de dioxyde de carbone mis en oeuvre au cours de l'étape de méthanation de dioxyde de carbone.

Dans des variantes, une étape de sortie de l'étape de méthanation de dioxyde de carbone est conjointe à une étape de sortie de l'étape de méthanation de monoxyde de carbone.

Dans des variantes, le procédé 30 comporte, en aval de l'étape de méthanation de monoxyde de carbone et/ou de l'étape de combustion, une étape de condensation de l'eau contenu dans des vapeurs et pour alimenter l'étape d'électrolyse en eau.

## Revendications

1. Dispositif intégré de production de gaz naturel de substitution, **caractérisé en ce qu'**il comporte :
- un gazéificateur (102) configuré pour produire un composé gazeux à partir d'une biomasse, comportant :
- une entrée (104) pour la biomasse ;
- une entrée (106) pour un agent oxydant et
- une sortie (108) du composé gazeux comportant du monoxyde de carbone ;
- un premier moyen (110) de méthanation du monoxyde de carbone, disposé en aval du gazéificateur (102), pour produire un composé comportant du gaz naturel de substitution à partir du composé gazeux sortant du gazéificateur, le moyen (110) de méthanation du monoxyde de carbone comportant au moins une entrée (112) pour de l'eau et une entrée pour le composé gazeux issu du gazéificateur (102),
- en aval du premier moyen (110) de méthanation de monoxyde de carbone, un séparateur (146) de dioxyde de carbone pour alimenter un deuxième moyen (114) de méthanation de dioxyde de carbone en dioxyde de carbone,
- le deuxième moyen (114) de méthanation de dioxyde de carbone pour produire un composé comportant du gaz naturel de substitution comportant au moins une entrée (116) pour de l'eau et une entrée pour du dioxyde de carbone provenant du séparateur (146),
- un moyen (118) de production de dihydrogène à partir d'eau et de courant électrique comportant :
- une alimentation électrique,
- une entrée (120) pour eau et
- une sortie (122) pour dihydrogène alimentant le deuxième moyen (114) de méthanation de dioxyde de carbone.

2. Dispositif selon la revendication 1, qui comporte, de plus, un moyen (124) de combustion, comportant :
- une entrée (126) pour une partie solide résultant d'une pyrolyse d'un combustible solide, aussi appelée « char », non gazéifié en provenance du gazéificateur (102) et transporté par un média caloporteur,
- une entrée (128) pour comburant,
- un moyen (130) de combustion du char non gazéifié pour chauffer le média caloporteur,
- une sortie (132) du média caloporteur reliée à une entrée pour média caloporteur du gazéificateur (102) et
- une sortie (134) pour fumées.

3. Dispositif selon la revendication 2, dans lequel le moyen (118) de production de dihydrogène est configuré pour réaliser une électrolyse de l'eau comportant une sortie (136) pour dioxygène alimentant l'entrée (128) en comburant du moyen de combustion.

4. Dispositif selon l'une des revendications 2 ou 3, qui comporte, entre la sortie (108) de composé gazeux du gazéificateur (102) et l'entrée (112) pour composé gazeux du premier moyen (110) de méthanation de monoxyde de carbone, un séparateur (138) configuré pour séparer les gaz des solides et/ou goudrons dans le composé gazeux et pour transmettre les solides et/ou goudrons séparés au moyen (124) de combustion.

5. Dispositif selon l'une des revendications 2 à 4, qui comporte un moyen (140) de recyclage d'une partie de la fumée, comportant du dioxygène, sortant du moyen (124) de combustion vers une entrée (128) de comburant du moyen de combustion.

6. Dispositif selon l'une des revendications 2 à 5, qui comporte, en aval de la sortie (134) de fumée du moyen (124) de combustion, un séparateur (142) de dioxyde de carbone configuré pour alimenter le deuxième moyen (114) de méthanation de dioxyde de carbone en dioxyde de carbone.

7. Dispositif selon l'une des revendications 1 à 6, qui comporte un séparateur (144) de dihydrogène en aval du premier moyen (110) de méthanation de monoxyde de carbone pour alimenter ledit premier moyen (110) de méthanation de monoxyde de carbone en dihydrogène.

8. Dispositif selon l'une des revendications 1 à 7, dans lequel une sortie du composé comportant du gaz naturel de substitution du deuxième du moyen (114) de méthanation de dioxyde de carbone est reliée à une sortie du composé comportant du gaz naturel de substitution du premier moyen (110) de méthanation de monoxyde de carbone.

9. Dispositif selon l'une des revendications 1 à 8, qui comporte, en aval du moyen de (110) méthanation de monoxyde de carbone et/ou du moyen (124) de combustion, un condenseur (148) configuré pour condenser de l'eau contenu dans des vapeurs et pour alimenter le moyen (118) d'électrolyse en eau.

10. Réseau, **caractérisé en ce qu'**il comporte au moins un dispositif (205) selon l'une des revendications 1 à 9.

11. Réseau selon la revendication 10, qui comporte un moyen (210) de gestion multi-énergie pour commander :
- la production, avec au moins un dispositif (205) selon l'une des revendications 1 à 9, et le stockage du méthane au cours de durées de production d'électricité en excès et
- la production d'électricité avec le méthane stocké en dehors de ces durées.

12. Réseau selon la revendication 11, qui comporte des canalisations (215) de distribution de gaz, le stockage du méthane pour générer de l'électricité étant réalisé par surpression au-delà de la pression nominale des canalisations.

13. Procédé de production de gaz naturel de substitution, **caractérisé en ce qu'**il comporte :
- une étape de gazéification (305) pour produire un composé gazeux à partir d'une biomasse, comportant :
- une étape d'entrée (310) de la biomasse ;
- une étape d'entrée (315) d'un agent oxydant et
- une étape de sortie (320) du composé gazeux comportant du monoxyde de carbone ;
- une première étape (325) de méthanation du monoxyde de carbone, en aval de l'étape de gazéification, pour produire un composé comportant du gaz naturel de substitution à partir du composé gazeux issu de l'étape de gazéification, l'étape (325) de méthanation du monoxyde de carbone comportant au moins une étape d'entrée (330) pour de l'eau et pour le composé gazeux issu du gazéificateur,
- en aval de la première étape de méthanation de monoxyde de carbone, une étape de séparation de dioxyde de carbone pour alimenter une deuxième étape de méthanation de dioxyde de carbone en dioxyde de carbone,
- une deuxième étape (340) de méthanation de dioxyde de carbone pour produire un composé comportant du gaz naturel de substitution comportant au moins une étape d'entrée (345) pour de l'eau et une entrée pour du dioxyde de carbone provenant de l'étape de séparation,
- une étape (355) de production de dihydrogène à partir d'eau et de courant électrique comportant :
- une étape (370) d'alimentation électrique,
- une étape (360) d'entrée pour eau et
- une étape (365) de sortie pour dihydrogène utilisé au cours de la deuxième étape de méthanation de dioxyde de carbone.

14. Procédé selon la revendication 13, qui comporte, de plus, une étape de combustion, comportant :
- une étape d'entrée entrée pour une partie solide résultant d'une pyrolyse d'un combustible solide, aussi appelée « char », non gazéifié en provenance du gazéificateur et transporté par un média caloporteur,
- une étape d'entrée pour comburant,
- une étape de combustion du char non gazéifié pour chauffer le média caloporteur,
- une étape de sortie du média caloporteur reliée à une entrée pour média caloporteur du gazéificateur et
- une étape de sortie pour fumées.

## Patentansprüche

1. Integrierte Produktionsvorrichtung von Substitutions-Naturgas, **dadurch gekennzeichnet, dass** sie umfasst:
- eine Vergasungsanlage (102), die konfiguriert ist, um einen gashaltigen Bestandteil ausgehend von einer Biomasse zu produzieren, umfassend:
- einen Eingang (104) für die Biomasse;
- einen Eingang (106) für ein Oxidationsmittel und
- einen Ausgang (108) des gashaltigen Bestandteils, umfassend Kohlenmonoxid;
- ein erstes Mittel (110) zur Methanisierung des Kohlenmonoxids, das der Vergasungsanlage (102) nachgeschaltet ist, um einen Substitutions-Naturgas umfassenden Bestandteil ausgehend von dem aus der Vergasungsanlage austretenden gashalten Bestandteil zu produzieren, wobei das Mittel (110) zur Methanisierung von Kohlenmonoxids wenigstens einen Eingang (112) für Wasser und einen Eingang für den aus der (102) Vergasungsanlage stammenden gashalten Bestandteil umfasst,
- dem ersten Mittel (110) zur Methanisierung von Kohlenmonoxid nachgeschaltet einen Kohlendioxidabscheider (146) zur Versorgung eines zweiten Mittels (114) zur Methanisierung von Kohlendioxid in Kohlendioxid,
- wobei das zweite Mittel (114) zur Methanisierung von Kohlendioxid zum Produzieren eines Substitutions-Naturgas umfassenden Bestandteils wenigstens einen Eingang (116) für Wasser und einen Eingang für aus dem Abscheider (146) stammende Kohlendioxid umfasst,
- ein Mittel (118) zum Produzieren von Dihydrogen ausgehend von Wasser und elektrischem Strom, umfassend:
- eine Stromversorgung,
- einen Eingang (120) für Wasser und
- einen Ausgang (122) für Dihydrogen, der das zweite Mittel (114) zur Methanisierung von Kohlendioxid versorgt.

2. Vorrichtung gemäß Anspruch 1, die darüber hinaus ein Verbrennungsmittel (124) umfasst, umfassend:
- einen Eingang (126) für einen festen Teil, der aus einer Pyrolyse eines festen Brennstoffs resultiert, ebenfalls bezeichnet als "Wagen", nicht in Gas umgewandelt ist, aus der Vergasungsanlage (102) stammt und durch ein Wärmeüberträger-Medium transportiert ist,
- einen Eingang (128) für einen Sauerstoffträger,
- ein Verbrennungsmittel (130) des nicht in Gas umgewandelten Wagens zum Erhitzen des Wärmeüberträger-Mediums,
- einen Ausgang (132) des Wärmeüberträger-Mediums, der mit einem Eingang für das Wärmeüberträger-Medium des Vergasungsmittels (102) verbunden ist, und
- einen Ausgang (134) für Rauch.

3. Vorrichtung gemäß Anspruch 2, bei dem das Mittel (118) zur Produktion von Dihydrogen konfiguriert ist, um eine Elektrolyse des Wassers zu realisieren, umfassend einen Ausgang (136) für Disauerstoff, der den Eingang (128) mit dem Sauerstoffträger des Verbrennungsmittels versorgt.

4. Vorrichtung gemäß einem der Ansprüche 2 oder 3, die zwischen dem Ausgang (108) des gashaltigen Bestandteils der Vergasungsanlage (102) und dem Eingang (112) für dem gashaltigen Bestandteil des ersten Mittels (110) zur Methanisierung von Kohlenmonoxid einen Abscheider (138) umfasst, der konfiguriert ist, um die Gase der Feststoffe und / oder Teere in dem gashaltigen Bestandteil zu trennen und um die in dem Verbrennungsmittel (124) abgeschiedenen Feststoffe und / oder Teere zu trennen.

5. Vorrichtung gemäß einem der Ansprüche 2 bis 4, die ein Mittel (140) zum Recyceln eines Teils des Rauchs umfasst, umfassend Disauerstoff, der aus dem Verbrennungsmittel (124) zu einem Eingang (128) des Sauerstoffträgers des Verbrennungsmittels austritt.

6. Vorrichtung gemäß einem der Ansprüche 2 bis 5, die dem Ausgang (134) für den Rauch des Verbrennungsmittels (124) nachgeschaltet einen Kohlendioxid-Abscheider (142) umfasst, der konfiguriert ist, um das zweite Mittel (114) zur Methanisierung von Kohlendioxid mit Kohlendioxid zu versorgen.

7. Vorrichtung gemäß einem der Ansprüche 1 bis 6, die einen Dihydrogen-Abscheider (144) umfasst, der dem ersten Mittel (110) zur Methanisierung von Kohlenmonoxid nachgeschaltet ist, um das genannte erste Mittel (110) zur Methanisierung von Kohlenmonoxid mit Dihydrogen zu versorgen.

8. Vorrichtung gemäß einem der Ansprüche 1 bis 7, bei dem ein Ausgang des Bestandteils, der Substitutionsgas des zweiten Mittels (114) zur Methanisierung von Kohlendioxid umfasst, mit einem Ausgang des Bestandteils verbunden ist, der Substitutionsgas des ersten Mittels (110) zur Methanisierung von Kohlenmonoxid umfasst.

9. Vorrichtung gemäß einem der Ansprüche 1 bis 8, die dem Mittel (110) zur Methanisierung von Kohlenmonoxid und / oder Verbrennungsmittel (124) nachgeschaltet einen Kondensator (148) umfasst, der konfiguriert ist, um das in den Dämpfen enthaltene Wasser zu kondensieren und um das Elektrolysemittel (118) mit Wasser zu versorgen.

10. Netz, **dadurch gekennzeichnet, dass** es wenigstens eine Vorrichtung (205) gemäß einem der Ansprüche 1 bis 9 umfasst.

11. Netz gemäß Anspruch 10, das ein Multi-Energieverwaltungsmittel (210) umfasst, um zu steuern:
- die Produktion mit wenigstens einer Vorrichtung (205) gemäß einem der Ansprüche 1 bis 9, und die Speicherung des Methans im Verlauf von Produktionsdauern von überschüssigem Strom und
- die Produktion von Strom mit dem außerhalb dieser Dauern gespeicherten Methan.

12. Netz gemäß Anspruch 11, das Kanalisationen (215) zur Gasverteilung umfasst, wobei die Speicherung des Methans zum Generieren des Stroms per Überdruck über den Nenndruck der Kanalisationen hinaus realisiert ist.

13. Produktionsverfahren von Substitutions-Naturgas, **dadurch gekennzeichnet, dass** es umfasst:
- einen Vergasungsschritt (305) zum Produzieren eines gashaltigen Bestandteils ausgehend von einer Biomasse, umfassend:
- einen Eingangsschritt (310) der Biomasse;
- einen Eingangsschritt (315) eines Oxidationsmittels und
- einen Ausgangsschritt (320) des Kohlenmonoxid umfassenden gashaltigen Bestandteils;
- einen ersten Schritt (325) zur Methanisierung des Kohlenmonoxids nach dem Vergasungsschritt, um einen Substitutions-Naturgans umfassenden Bestandteil ausgehend von dem gashaltigen Bestandteil zu produzieren, der aus dem Vergasungsschritt stammt, wobei der Schritt (325) zur Methanisierung des Kohlenmonoxids wenigstens einen Eingangsschritt (330) für Wasser und für den aus der Vergasungsanlage stammenden gashaltigen Bestandteil umfasst,
- dem ersten Schritt zur Methanisierung von Kohlenmonoxid nachgeschaltet einen Abscheidungsschritt von Kohlendioxid, um einen zweiten Methanisierungsschritt von Kohlendioxid mit Kohlendioxid zu versorgen,
- einen zweiten Schritt (340) zur Methanisierung von Kohlendioxid zum Produzieren eines Bestandteils, umfassend Substitutions-Naturgas umfassend wenigstens einen Eingangsschritt (345) für Wasser und einen Eingang für aus dem Abscheidungsschritt stammendes Kohlendioxid,
- einen Schritt (355) zur Produktion von Dihydrogen ausgehend von Wasser und elektrischem Strom, umfassend:
- einen Schritt (370) zur elektrischen Versorgung,
- einen Eingangsschritt (360) für Wasser und
- einen Ausgangsschritt (350) für Dihydrogen, das im Verlauf des zweiten Schritts zur Methanisierung von Kohlendioxyd verwendet wird.

14. Verfahren gemäß Anspruch 13, das darüber hinaus einen Verbrennungsschritt umfasst, umfassend:
- einen Eingangsschritt für einen festen Teil, der aus einer Pyrolyse eines festen Brennstoffs resultiert, ebenfalls bezeichnet als "Wagen", nicht in Gas umgewandelt, aus der Gasanlage stammend und durch ein Wärmeträger-Medium transportiert ist,
- einen Eingangsschritt für einen Sauerstoffträger,
- einen Verbrennungsschritt des nicht in Gas umgewandelten Wagens zum Heizen des Wärmeträger-Mediums,
- einen Ausgangsschritt des Wärmeträger-Mediums, der mit einem Eingang für ein Wärmeträger-Medium der Vergasungsanlage verbunden ist, und
- einen Ausgangsschritt für Rauch.

## Claims

1. Integrated device for producing substitute natural gas, comprising:
- a gasifier (102) configured for producing a gaseous compound from a biomass, comprising:
- an inlet (104) for biomass;
- an inlet (106) for an oxidizing agent; and
- an outlet (108) for the gaseous compound comprising carbon monoxide;
- a first means (110) for methanating the carbon monoxide, downstream from the gasifier (102), to produce substitute natural gas from the gaseous compound output from the gasifier, the carbon monoxide methanation means (110) comprising at least one inlet (112) for water and an inlet for the gaseous compound coming from the gasifier;
- downstream from the first means (110) for methanating, a carbon dioxide separator (146) for supplying a second means for methanating carbon dioxide in carbon dioxide,
- the second means (114) for methanating carbon dioxide to produce a gas compound comprising substitute natural gas comprising at least one inlet (116) for water and an inlet for the carbon dioxide coming from the separator (146);
- a means (118) for producing dihydrogen from water and electric current comprising:
- an electrical power supply;
- an inlet (120) for water and
- an outlet (122) for dihydrogen supplying the means for methanating carbon dioxide (118).

2. Device according to claim 1, that also comprises a combustion means (124), comprising:
- an inlet (126) for a solid portion resulting from pyrolysis of a non-gasified solid combustible, also called "char", coming from the gasifier (102) and transported by a heat transfer medium;
- an oxidizer inlet (128);
- a non-gasified char combustion means (130) for heating the heat transfer medium;
- a heat transfer medium outlet (132) linked to a heat transfer medium inlet of the gasifier (102) and
- an outlet (134) for flue-gases.

3. Device according to claim 2, wherein the dihydrogen production means (118) is configured to carry out an electrolysis of water, comprising a dioxygen outlet (136) supplying the oxidizer inlet (128) of the combustion means.

4. Device according to one of claims 2 or 3, that comprises between the gaseous compound outlet (108) of the gasifier (102) and the gaseous compound inlet (112) of the first carbon monoxide methanation means (110), a separator (138) configured to separate the gases from the solids and/or tars in the gaseous compound and to transmit the separated solids and/or tars to the combustion means (124).

5. Device according to one of claims 2 to 4, that comprises a means (140) of recycling a portion of the flue-gas, on output from the combustion means (124), comprising dioxygen, towards an oxidizer inlet (128) of the combustion means.

6. Device according to one of claims 2 to 5, that comprises, downstream from the flue-gas outlet (134) of the combustion means (124), a carbon dioxide separator (142) configured to supply the carbon dioxide methanation means (114) with carbon dioxide.

7. Device according to one of claims 1 to 6, that comprises a dihydrogen separator (144) downstream from the carbon monoxide methanation means (110) in order to supply said carbon monoxide methanation means (110) with dihydrogen.

8. Device according to one of claims 1 to 7, wherein an outlet of gas compound comprising substitute natural gas from the second carbon dioxide methanation means (114) is linked to an outlet of gas compound comprising substitute natural gas from the first carbon monoxide methanation means (110).

9. Device according to one of claims 1 to 8, that comprises, downstream from the carbon monoxide methanation means (110) and/or from the combustion means (124), a condenser (148) configured to condense the water contained in vapors and to supply the electrolysis means (118) with water.

10. Network, **characterized in that** it comprises at least one device (205) according to one of claims 1 to 9.

11. Network according to claim 12, that comprises a multi-energy management means (210) for controlling:
- the production, with at least one device (205) according to one of claims 1 to 9, and storage of methane during periods of surplus electricity production; and
- the production of electricity with the stored methane outside these periods.

12. Network according to claim 11, that comprises gas distribution pipelines (215), the storage of methane for generating electricity being realized by overpressure above the nominal pressure of the pipelines.

13. Method for producing substitute natural gas, comprising:
- a gasification step (305) for producing a gaseous compound from a biomass, comprising:
- a step of inputting (310) the biomass;
- a step of inputting (315) an oxidizing agent; and
- a step of outputting (320) the gaseous compound comprising carbon monoxide;
- a first step (325) of methanating the carbon monoxide to produce a gaseous compound comprising substitute natural gas from the gaseous compound output from the gasification step, the carbon monoxide methanation step (325) comprising at least one input step (330) for water and for the gaseous compound from the gasifier;
- downstream from the first step of mathanating the carbon monoxide, a step of separating carbon monoxide to supply a second means for methanating carbon dioxide in carbon dioxide,
- a second step (340) of methanating carbon dioxide to produce gas compound comprising substitute natural gas comprising at least one input step (345) for water and an input for carbon dioxide coming from the carbon monoxide methanation step;
- a step (355) of producing dihydrogen from water and electric current comprising:
- a step (370) of supplying electrical power;
- a step (360) of inputting water, and
- an output step (365) for dihydrogen used during the second carbon dioxide methanation step.

14. Method according to claim 13, that also comprises a combustion step, comprising:
- a step of inputting a solid portion resulting from pyrolysis of a non-gasified solid combustible, also called "char", coming from the gasifier and transported by a heat transfer medium;
- an oxidizer input step;
- a non-gasified char combustion step for heating the heat transfer medium;
- a heat transfer medium output step linked to an input of heat transfer medium for the gasifier; and
- a step of outputting flue-gases.
